(19) Europäisches Patentamt

European Patent Office

Office européen des brevets

(11) **EP 1 443 449 A2**

(12) **EUROPEAN PATENT APPLICATION**

(43) Date of publication:
**04.08.2004 Bulletin 2004/32**

(51) Int Cl.⁷: **G06F 19/00**

(21) Application number: **04002314.5**

(22) Date of filing: **03.02.2004**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **03.02.2003 KR 2003006543**
**30.01.2004 KR 2004005945**

(71) Applicant: **Samsung Electronics Co., Ltd.**
**Suwon-si, Gyeonggi-do (KR)**

(72) Inventor: **Ahn, Tae-jin**
**Seoul (KR)**

(74) Representative: **Grünecker, Kinkeldey,**
**Stockmair & Schwanhäusser Anwaltssozietät**
**Maximilianstrasse 58**
**80538 München (DE)**

(54) **Apparatus, method and computer readable medium for encoding a DNA sequence**

(57)     An apparatus and a method for encoding a DNA sequence are provided. A comparative unit aligns a reference sequence having known DNA information with a subject sequence to be encoded so that consensus bases of the two sequences are optimally matched and extracts a difference between the two sequences. A conversion unit converts information of the extracted difference between the reference sequence and the subject sequence into a string of predetermined characters. An encoding unit encodes the individual characters that make the string of the characters using predetermined conversion codes corresponding to the individual characters stored in a code storage unit. A compression unit compresses the encoded result using a common compression method. The compressed result is stored in a sequence storage unit.

# FIG. 1

**Description**

BACKGROUND OF THE INVENTION

1. Field of the Invention

The present invention relates to an apparatus and a method for encoding a DNA sequence. More particularly, the present invention relates to an apparatus and a method for encoding a DNA sequence capable of decreasing storage space and transfer traffic through more efficient compression and providing security during storage and transfer of the DNA sequence.

2. Description of the Related Art

With development of the biotechnology, a DNA sequence that contains specific genetic information of an organism has been analyzed and revealed. Such a DNA sequence analysis can be applied to various purposes such as finding genetic factors that cause the phenotypic variations and diseases of organisms and is actively performed with the aid of a computer. In this regard, it is necessary to convert a DNA sequence into a computer readable form. However, since a DNA sequence contains bulky genetic information and a need for storage of a DNA sequence is increasing, enormous cost for its storage and transfer is incurred. Therefore, in order to ensure the storage, transfer, and search of a DNA sequence, compression of the DNA sequence is required.

A compression method for a DNA sequence is largely classified into dictionary based and non-dictionary based. The dictionary based compression method achieves a high compression ratio. According to this compression method, a compression ratio is generally equal to 70 to 80%. However, This compression method cannot be applied in compression of a whole genomic DNA sequence.

The best current DNA sequence compression strategy can achieve compression of a whole genome. According to this strategy, it is reported that a compression ratio is generally equal to 70 to 80%, and the genome of *E. coli* is compressed at a compression ratio of 96.6%. However, these compression ratios are simply presumptive values and no specific approaches for achieving these compression ratios are disclosed.

SUMMARY OF THE INVENTION

The present invention provides an apparatus and a method for encoding a DNA sequence capable of decreasing storage space and transfer traffic through efficient compression and providing security during storage and transfer of the DNA sequence.

The present invention also provides a computer readable medium having embodied thereon a computer program for a method for encoding a DNA sequence capable of decreasing storage space and transfer traffic through efficient compression and providing security during storage and transfer of the DNA sequence.

According to an aspect of the present invention, there is provided an apparatus for encoding a DNA sequence, which comprises: a comparative unit aligning a reference sequence having known DNA information with a subject sequence to be encoded and extracting a difference between the reference sequence and the subject sequence; a conversion unit converting information of the extracted difference between the reference sequence and the subject sequence into a string of predetermined characters; a code storage unit storing predetermined conversion codes that correspond to the individual characters; and an encoding unit encoding the individual characters that make the string of the characters using the conversion codes.

According to another aspect of the present invention, there is provided a method for encoding a DNA sequence, which comprises: aligning a reference sequence having known DNA information with a subject sequence to be encoded; extracting a difference between the reference sequence and the subject sequence; converting information of the extracted difference between the reference sequence and the subject sequence into a string of predetermined characters; and coding the individual characters that make the string of the characters using predetermined conversion codes that correspond to the individual characters.

Therefore, a DNA sequence can be stored at a compression ratio of 90% or more without loss of genetic information, and high security is ensured. Furthermore, such a high compression ratio is efficient to store a genome sequence or multiple DNA sequences for a specific region of a genome.

BRIEF DESCRIPTION OF THE DRAWINGS

The above and other features and advantages of the present invention will become more apparent by describing in detail exemplary embodiments thereof with reference to the attached drawings in which:

FIG. 1 is a block diagram showing the structure of an apparatus for encoding a DNA sequence according to an embodiment of the present invention;

FIG. 2 is a view that illustrates the comparison result of a reference DNA sequence and a subject DNA sequence using NCBI's blast;

FIG. 3 is a view that illustrates a principle of conversion of information about a difference between a reference DNA sequence and a subject DNA sequence that are aligned in a comparative unit into a string of characters;

FIG. 4 is a view that illustrates 4 bit codes for encoding a string of characters;

FIG. 5 is a view that illustrates conversion of the exons of mody3 gene into a string of characters and 4-bit encoding of the string of the characters;

FIG. 6 is a flow diagram showing a process for encoding a DNA sequence according to an embodiment of the present invention;

FIG. 7 is a block diagram showing the structure of an apparatus for encoding a DNA sequence according to another embodiment of the present invention;

FIG. 8 is a view that illustrates a process of modifying a reference sequence according to variation sequence induction factors presented in Table 2; and

FIG. 9 is a flow diagram showing a process for encoding a DNA sequence according to another embodiment of the present invention.

DETAILED DESCRIPTION OF THE INVENTION

Hereinafter, an apparatus and a method for encoding a DNA sequence according to the present invention will be described in more detail with reference to the accompanying drawings.

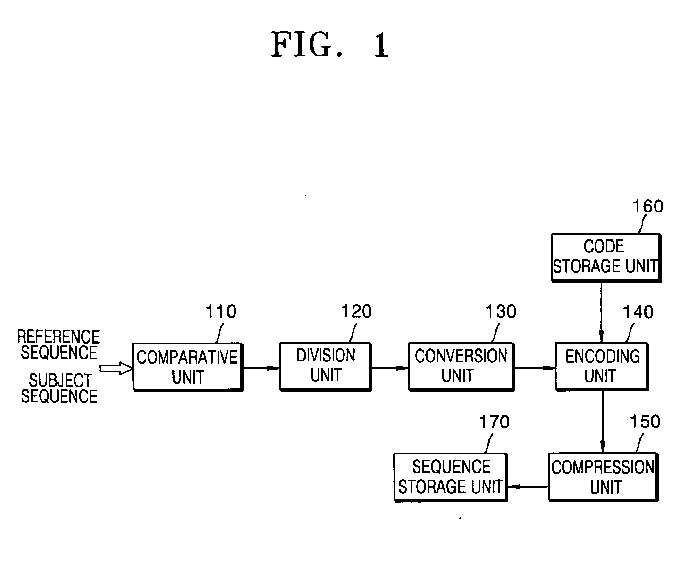

FIG. 1 is a block diagram that illustrates the structure of an apparatus for encoding a DNA sequence according to an embodiment of the present invention.

Referring to FIG. 1, an apparatus 100 for encoding a DNA sequence includes a comparative unit 110, a division unit 120, a conversion unit 130, an encoding unit 140, a compression unit 150, a code storage unit 160, and a sequence storage unit 170.

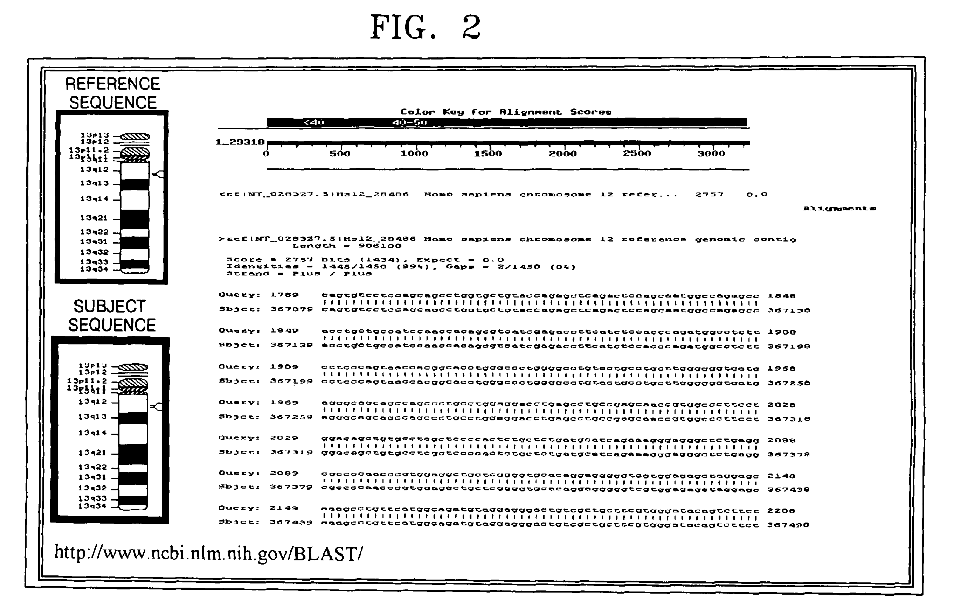

The comparative unit 110 aligns a subject sequence to be encoded with a reference sequence, of which DNA information is known, to extract a difference between the two sequences. In this case, the reference sequence and the subject sequence are aligned so that consensus bases are optimally matched. The division unit 120 divides the extracted difference between the reference sequence and the subject sequence into segments of predetermined sizes. Preferably, such division is carried out so that each segment size is equal to 15% of the whole capacity of the sequence storage unit 170. FIG. 2 shows the comparison result of the reference DNA sequence and the subject DNA sequence using NCBI's blast. The comparison result can be output in a document format such as text, html, or xml. A known parsing method enables to extraction of only the difference between the reference sequence and the subject sequence from the comparison result.

The conversion unit 130 converts information of the extracted difference between the reference sequence and the subject sequence into a string of 16 characters. The difference between the reference sequence and the subject sequence may be classified into six patterns. In the conversion unit 130, the six patterns are expressed as a string of 16 characters. These 16 characters include ten numeric characters for 0 through 9, four DNA symbols for A, T, G, and C, and two identifiers for discerning information. Table 1 presents the 16 characters for expressing differences between the reference sequence and the subject sequence and the descriptions thereof.

Table 1

| Characters | Descriptions | |
|---|---|---|
| A | Adenine | DNA symbols of subject sequence different from reference sequence |
| T | Thymine | |
| G | Guanine | |
| C | Cytocine | |
| 0-9 | Numeric characters for expressing start position, continued length, and distance between start position and end position of differences | |
| / | Identifier for expressing the starting and ending of differences | |
| ~ | Identifier for expressing the continuation of differences | |

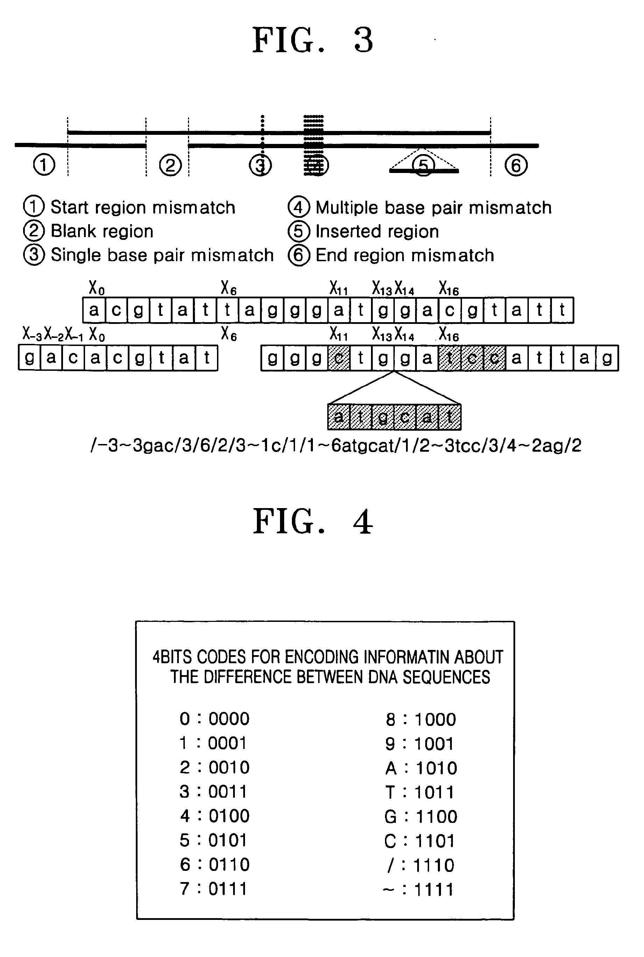

A principle for converting differences between the reference sequence and the subject sequence into a string of characters will now be described with reference to FIG. 3. However, the conversion principle of FIG. 3 is provided only for illustration and thus the present invention is not limited to or by them.

First, the patterns of differences between the reference sequence and the subject sequence are analyzed.

A. Start region mismatch: the start region ranging from $X_{-3}$ to $X_{-1}$ of the subject sequence is not present on the reference sequence and corresponds to gac sequence.

B. Blank: the region ranging from $X_6$ to $X_7$ of the reference sequence is not present on the subject sequence and corresponds to ta sequence.

C. Single base pair mismatch: at the region of $X_{11}$, the DNA base of the reference sequence is different from that of the subject sequence.

D. Insertion: atgcat sequence absent on the reference sequence is present between $X_{13}$ and $X_{14}$ of the subject sequence.

E. Multiple base pair mismatch: at the regions of $X_{16}$ to $X_{18}$, the DNA bases of the reference sequence are different from those of the subject sequence.

F. End region mismatch: the end region ranging from $X_{22}$ to $X_{23}$ of the subject sequence is not present on the reference sequence and corresponds to ag sequence.

Next, the above-described difference patterns are sequentially converted into characters.

The pattern of A is converted into "/-3~3gac/3" characters. Here, the first "/" represents the starting of the A pattern. The "-3" represents the start position of the A pattern, i.e., the position 3 upstream from the origin, $X_0$. The "~" represents the continuation of the A pattern. The first "3" represents the continued length of the A pattern. The "gac" represents the starting DNA bases of the subject sequence different from the reference sequence. The second "/" represents the ending of the A pattern. The second "3" represents the distance between the start position and the end position of the A pattern.

The pattern of B is converted into "/6/2" characters. Here, the "/6" represents the starting of the B pattern at the position $X_6$ that is 6 bases downstream from the $X_0$, a position which is determined by the "3" that represents the distance between the start position and the end position of the A pattern. The "2" represents the distance between the start position and the end position of the B pattern.

The pattern of C is converted into "/3~1c/1" characters. Here, the "/3" represents the starting of the C pattern at the position $X_{11}$ that is 3 bases downstream from $X_8$, a position which is determined by the "2" that represents the distance between the start position and the end position of the B pattern. The "~1" represents that the number of the continued bases of the C pattern is one. The "c" represents the DNA base of the subject sequence different from the reference sequence. The "1" represents the distance between the start position and the end position of the C pattern.

The pattern of D is converted into "/1~6atgcat/1" characters. Here, the "/1" represents the starting of the D pattern at the position $X_{13}$ that is 1 base downstream from $X_{12}$, a position which is determined by the "1" that represents the distance between the start position and the end position of the C pattern. The "~6" represents that the number of the continued bases of the D pattern is six. The "atgcat" represents the DNA bases of the subject sequence different from the reference sequence. The last "1" represents the distance between the start position ($X_{13}$) and the end position of the D pattern. The distance "1" means the insertion of the DNA sequence.

The pattern of E is converted into "/2~3tcc/3" characters. Here, the "/2" represents the starting of the E pattern at the position $X_{16}$ that is 2 bases downstream from $X_{14}$, a position which is determined by the "1" that represents the distance between the start position and the end position of the D pattern. The "~3" represents that the number of the continued bases of the E pattern is three. The "tcc" represents the DNA bases of the subject sequence different from the reference sequence. The last "3" represents the distance between the start position ($X_{16}$) and the end position of the E pattern.

The pattern of F is converted into "/3~ag/2" characters. Here, the "/3" represents the starting of the F pattern at the position $X_{22}$ that is 3 bases downstream from $X_{19}$, a position which is determined by the "3" that represents the distance between the start position and the end position of the E pattern. The "~2" characters represent that the number of the continued bases of the F pattern is two. The "ag" represents the DNA bases of the subject sequence different from the reference sequence. The last "2" represents the distance between the start position ($X_{22}$) and the end position of the F pattern.

Based on the above descriptions, the subject sequence is expressed by a string of characters as follows. Since one byte equals one character, the total size of the string of the characters is 50 bytes.

"/-3~3gac/3/6/2/3~1c/1/1~6atgcat/1/2~3tcc/3/3~2ag/2"

The encoding unit 140 encodes the individual characters that make the string of the characters using 4 bit codes stored in the code storage unit 160. An example of the codes stored in the code storage unit 160 is shown in FIG. 4. The 4-bit encoding results for the individual strings of the characters for the patterns of FIG. 3 are as follows.

/-3~3gac/3: 1110000000000011111100111100101011011110011

/6/2: 1110011011100010

/3~1c/1: 11100011111000111011110001

/1~6atgcat/1: 1110011011111010101111001101101011011100001

/2~3tcc/3: 11100010111100111011110111011110011

/3~2ag/2: 1110001111110010101011001110010

Therefore, the final encoded result output from the encoding unit 140 is as follows. The total size is 25 bytes.

1110000000000011111100111100101011011110001111100110111000101110001111110001110111100001111001101111101010111100110110101101111000011110001011110011101111011101111000111110001111110010101011001110010

The compression unit 150 compresses the encoded result using a common compression method. The compression result is stored in the sequence storage unit 170.

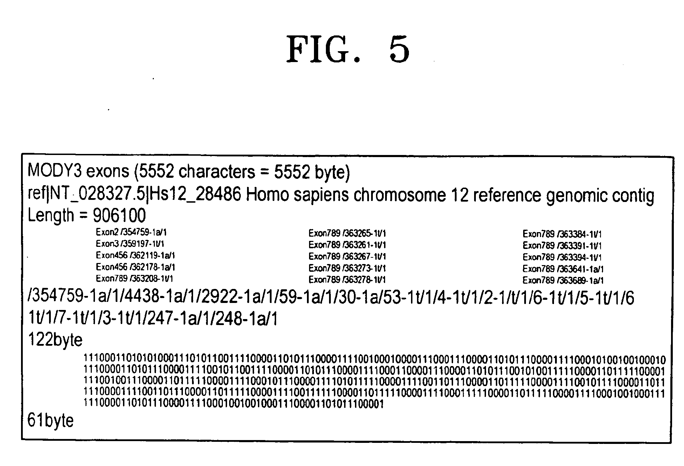

When conversion of differences between a reference sequence and a subject sequence into a string of characters and 4-bit encoding for the string of the characters are applied to the exons of the mody3 gene, a compression ratio of 98.9% or more can be obtained. Further, when the encoded exons of the mody3 gene are compressed, a higher compression ratio is obtained. FIG. 5 shows the results of conversion of the exons of the mody3 gene into a string of characters and 4-bit encoding of the string of the characters. Referring to FIG. 5, the exons of the mody3 gene with the size of 5552 bytes are converted into a string of characters of 122 bytes and then encoded into a string of codes of 61 bytes. A compression ratio is equal to 98.9%.

Meanwhile, a DNA sequence encoding apparatus according to the present invention may further include a pre-processing unit to support various coding format over same DNA sequence. The pre-processing unit acts as an encryption means of DNA sequence. In general, before a coded DNA sequence is stored in a storage means, predetermined security and encryption policy is applied to the coded DNA sequence. However, a DNA sequence encoding apparatus according to the present invention is used to apply particular security and encryption policy to a DNA sequence. A DNA sequence encoding apparatus having pre-processing unit creates template DNA sequences, selects a DNA sequence that can be used as an encryption key from the created template DNA sequences, and then encodes an object DNA sequence to be encoded. To decode a DNA sequence encoded by an above-mentioned method, a decoding apparatus corresponding to the DNA sequence encoding apparatus having pre-processing unit is needed. Therefore, in case of ill-intentioned distribution or hacking of a secret key, a DNA sequence encoding method according to the present invention provides higher quality of security service than a conventional encryption method using standard encryption algorithm with secret key.

An encoding method for a DNA sequence according to the present invention can be realized in common computing systems used in bioinformatics, such as personal computers (PCs), workstations, and super computers. The encoding and compression method for a known genomic DNA sequence of an organism can be divided into six steps.

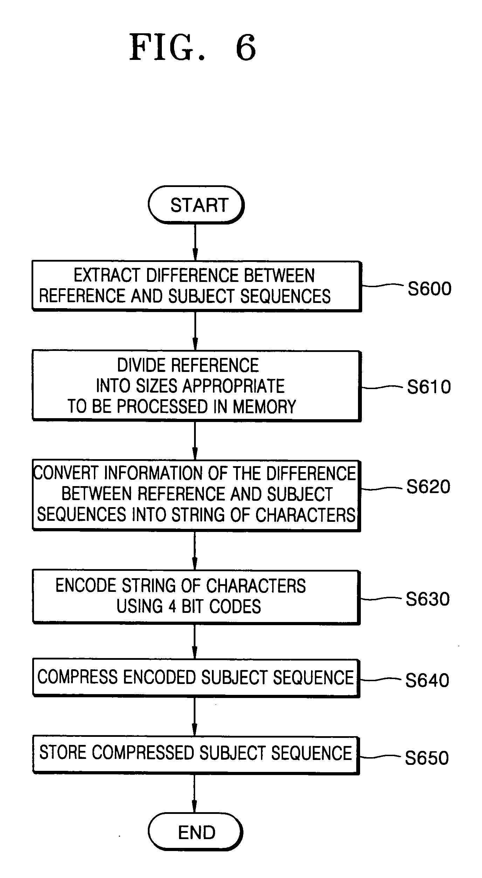

FIG. 6 is a flow diagram showing a DNA sequence encoding method according to an embodiment of the present invention.

Referring to FIG. 6, a difference between a known reference sequence and a subject sequence of an organism to be stored is extracted (step S600). The sequence comparison in step S600 may be carried out using conventional sequence homology search systems well known in the bioinformatics. Examples of sequence homology search systems that can be used herein include Blast, Blat, Fasta, and Smith-Waterman Algorithm. According to any one of the systems, the reference sequence and the subject sequence are aligned and compared. Output files are parsed by a known parsing technology to obtain the difference. Since it is an object of the present invention to encode only the difference between the two DNA sequences, it is important to align the two DNA sequences so that consensus bases of the two DNA sequences are optimally matched.

Next, an output file of step S600 is divided into segments of sizes appropriate to be processed in a memory (step S610). Since the whole genome sequence is several hundred megabytes in size, it is not preferable to encode the entire output file at a time. In this regard, the result of the aligning and the comparison is divided into segments of sizes each corresponding to 15% of the whole memory of the DNA sequence encoding apparatus according to the present invention.

Next, information of the difference between the reference sequence and the subject sequence is converted into a string of characters (step S620). The difference between the reference sequence and the subject sequence can be classified into six patterns. In step S620, these six patterns are converted into a string of 16 characters. These 16 characters include ten numeric characters for 0 through 9, four DNA symbols for A, T, G, and C, and two identifiers for discerning information.

The six patterns include start region mismatch, blank, single base pair mismatch, multiple base pair mismatch, insertion, and end region mismatch, which are terminologies that can be easily understood by ordinary persons skilled in the art.

Combination of these 16 characters enables to expression of difference information, such as the positions, DNA sequences, and lengths of the six patterns, as a string of characters. The string of the characters can be restored to an original subject sequence without loss of sequence information by comparison with the reference sequence. Such restoration is accomplished by reversing the conversion of the subject DNA sequence into the string of the characters.

Next, the DNA sequence expressed as the string of the characters is encoded by 4 bit codes (step S630). The individual characters that make the string of the characters can be expressed into 4 bit codes.

Next, the 4-bit encoded result is compressed using a conventional compression algorithm (step S640). A compression algorithm that can be used herein may be a tool well known in the data compression field such as LZ78, Hoffman coding, and computing coding. Furthermore, various known compression technologies related to compression of genetic information may be used. The compressed DNA sequence is stored in various storage means such as a hard disk and a CD (step S650).

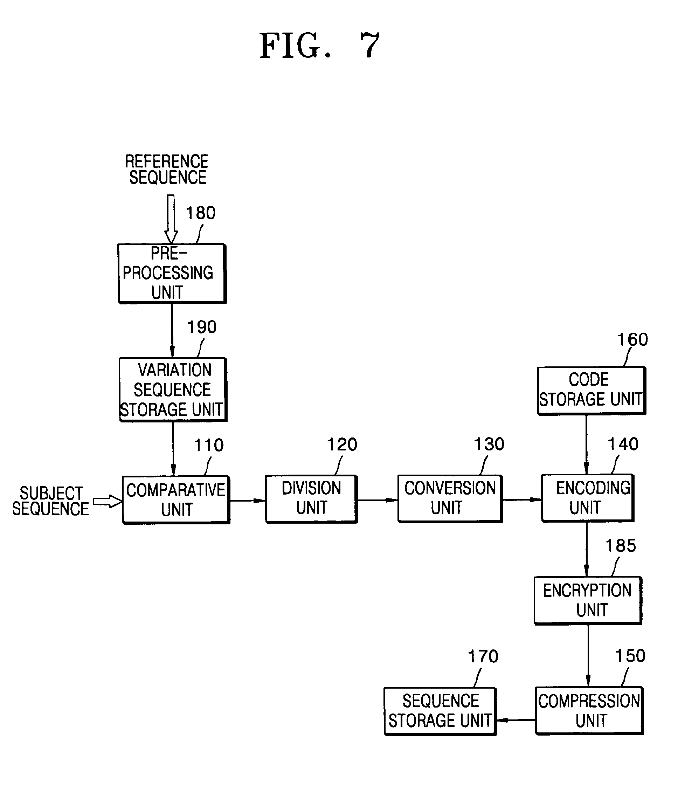

FIG. 7 is a block diagram showing the structure of an apparatus for encoding a DNA sequence according to another embodiment of the present invention. The remaining constitutional elements except a pre-processing unit 180, an encryption unit 185, and a variation sequence storage unit 190 in the DNA sequence encoding apparatus shown in FIG. 7 are the same as those in the embodiment described with reference to FIG. 1, and thus, the detailed descriptions thereof are omitted.

Referring to FIG. 7, the pre-processing unit 180 pre-processes a reference sequence for a DNA sequence to be encoded. The pre-process carried out in the pre-processing unit 180 is a type of encryption process of DNA sequence information. When the encryption unit 185 is further used, encoded DNA sequence information may be doubly encrypted. In this case, the encryption unit 185 encrypts DNA sequence information encoded by a DNA sequence encoding apparatus of the present invention according to an encryption algorithm well known prior to the filing of the present invention.

The pre-processing unit 180 pre-processes a reference sequence as follows. First, a variation sequence generation function for the reference sequence is created. The variation sequence generation function is a function that uses, as inputs, random variables that can be obtained by a technique embodied in computing science, for example, random number generation algorithm. Outputs (hereinafter, referred to as "variation sequence induction factors") of the variation sequence generation function include the total number of variations (TotalNv), a distance between variations (Nd), a length of variations (Lv), a type of variations (insertion/substitution), and a variation sequence (A, T, G, C, N: null). When the total number of variations is 4, an example of variation sequence generation factors for each of the variations is presented in Table 2 below. Here, "null" cannot be present together with another variation sequence. When "null" is present together with another variation sequence, it is present in the number that corresponds to the length of the variation sequence.

Table 2

| Section | Variation 1 | Variation 2 | Variation 3 | Variation 4 |
|---|---|---|---|---|
| Distance between variations | 1035 | 2220 | 3215 | 3200 |
| Length of variation | 1 | 4 | 7 | 5 |

Table 2 (continued)

| Section | Variation 1 | Variation 2 | Variation 3 | Variation 4 |
|---|---|---|---|---|
| Type of variation | Substitution | Substitution | Insertion | Substitution |
| Variation sequence | T | ATGG | ATGCGGG | NNNNN |

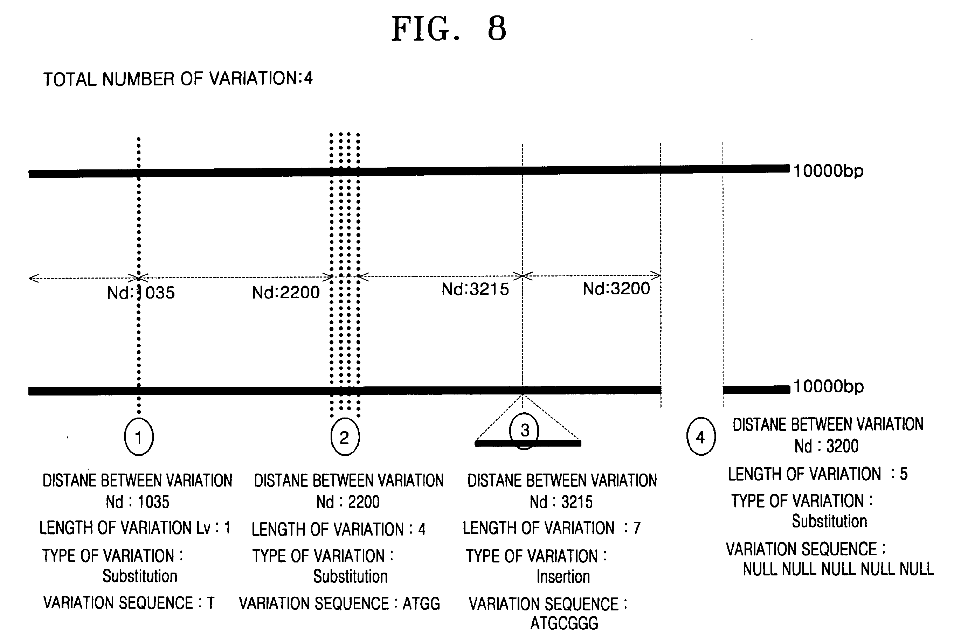

FIG. 8 is a view that illustrates a process of modifying a reference sequence according to variation sequence generation factors presented in Table 2. Referring to FIG. 8, the length of a reference sequence is 1,000 bp. Variation 1 that is a first variation is created at 1,035th bit downstream from the start position of the reference sequence. The length of the variation 1 is 1, the type of the variation 1 is substitution, and the sequence of the variation 1 is T. The pretreatment unit 180 modifies the reference sequence using some of the variation sequence generation factors output from the variation sequence generation function. That is, with respect to individual variation elements (variation 1, variation 2, variation 3, and variation 4), until queues of the variation elements are empty, predetermined variation sequences with predetermined lengths are substituted for or inserted in the reference sequence after distance shift corresponding to the distances between the variation elements. The variation sequences are stored in the variation sequence storage unit 190 and are input into a comparative unit 110 together with a subject sequence. In this case, the reference sequence and the selected variation sequence induction factors are separately stored as secret keys.

The DNA sequence encoding apparatus for security shown in FIG. 7 is different from that shown in FIG. 1 in terms of presence or absence of constitutional elements selecting a reference sequence. In a case where there exists one reference sequence for known species, and a DNA sequence is encoded based on the reference sequence, when the encoded DNA sequence is decoded in the absence of information on the reference sequence, the number of cases proportional to the length of the encoded DNA sequence is given. For example, in a case where a 100,000 bp long DNA sequence is encoded by the DNA sequence encoding apparatus of the present invention followed by compression, the number of cases when the encoded DNA sequence is decoded in the absence of information on a reference sequence is equal to the number of cases that selects reference sequences as many as the encoding length of a known genome sequence. Therefore, when a 100,000 bp of the human DNA sequence is encoded and compressed, the number of cases when the encoded human DNA sequence is decoded in the absence of information on a reference sequence is equal to (total length of the human DNA sequence - length of encoded human DNA sequence), i.e., (3.06 x $10^9$ - 100,000). In this regard, generally, in a case where after a n long DNA sequence is encoded, decoding of the encoded DNA sequence is carried out with all possible combinations in the absence of information on a reference sequence, the total number of cases is (3.06 x $10^9$ - n) and the probability is $1/(3.06 \times 10^9 - n)$. Therefore, encoding of a very long DNA sequence such as the whole genome sequence lowers security effect.

However, as described above, when a reference sequence is encoded after modified by the pretreatment unit, the security of a DNA sequence is enhanced. The pretreatment unit serves as encryption means using a secret key. Here, the secret key is a modified reference sequence and an encrypted document is a DNA sequence. According to the present invention, users can determine the degree of modification of a reference sequence according to security ranking. This means that users can control the number of secret keys to be created. That is, users can encrypt a DNA sequence using less or more secret keys than the number of secret keys that are used in an encryption algorithm such as triple-DES available commonly. The number of secret keys used in the triple-DES algorithm is $2^{168} \fallingdotseq 2.56 \times 10^{50}$. Meanwhile, the number ($N_{key}$) of secret keys that can be created in the DNA sequence encoding apparatus shown in FIG. 7 is as following Equation 1.

Equation 1

$$N_{key} = {_L}C_{TotalN_v} \times 2 \times (4 \times L_v + 1)$$

According to Equation 1, when the length of a reference sequence is 10,000 bp and the total number of variations is 16, secret keys of about $4.72 \times 10^{50}$ which is more than the number of the secret keys of triple-DES algorithm are created.

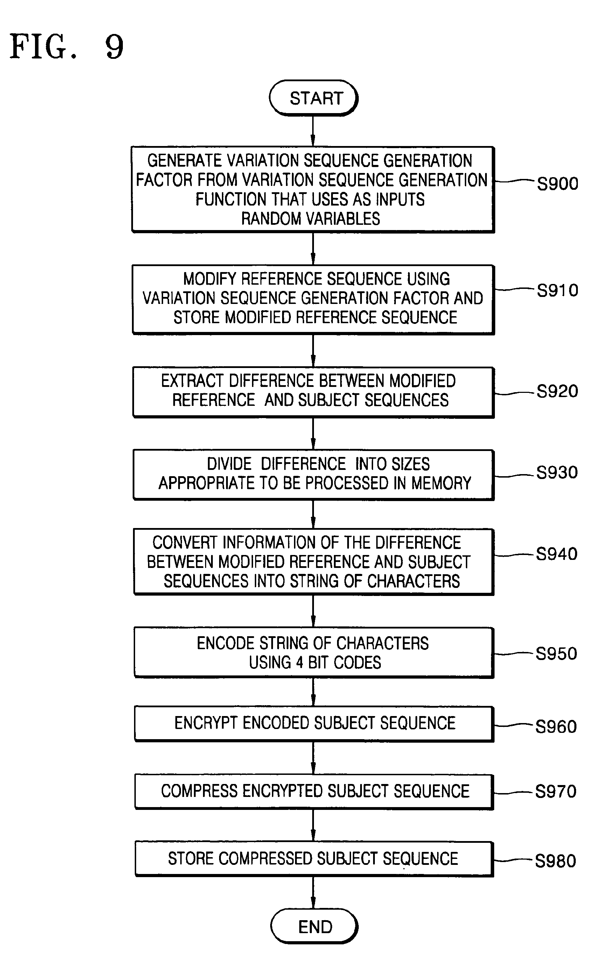

FIG. 9 is a flow diagram showing a DNA sequence encoding process that is carried out in the DNA sequence encoding apparatus shown in FIG. 7.

Referring to FIG. 9, the pre-processing unit 180 creates variation sequence generation factors from a variation sequence generation function that uses generated random variables as inputs (step S900). Also, the pre-processing unit 180 modifies a reference sequence using some of the created variation sequence generation factors and then stores the modified reference sequence in the variation sequence storage unit 190 (step S910). The comparative unit 110 extracts a difference between the modified reference sequence and a DNA sequence of an organism to be stored, i.e., a subject sequence (step S920). A division unit 120 divides the extracted difference into segments of sizes appropriate to be processed in a memory (step S930). A conversion unit 130 converts information of the difference between the reference sequence and the subject sequence into a string of characters (step S940). An encoding unit 140 encodes the individual characters that make the string of the characters using 4 bit codes (step S950). The encryption unit 185 encrypts the encoded DNA sequence using a common encryption algorithm (step S960). The encrypting by the encryption unit is optional. A compression unit 150 compresses the encrypted result using a common compression algorithm (step S970). The compressed DNA sequence is stored in a sequence storage unit 170 or transferred via a communication network (step S980).

According to the present invention, only the difference between a known reference sequence and a subject sequence is encoded and compressed. Therefore, homologies between the reference sequence and the subject sequence determine compression efficiency. According to a general biological knowledge, the same species have the sequence identity of 99% or more. In this regard, it can be said that only the difference of 1% or less is recorded. Therefore, when the present invention is applied in compression and storage of the human genome sequence, a compression ratio of 98.65% or more is expected.

Such a theoretical compression ratio of the human genome sequence can be explained under the following presumptions. These presumptions can be sufficiently accepted by ordinary persons skilled in the art. Generally, in the human genome, since a difference by blank or insertion little occurs, almost all differences might be caused by single base pair mismatch. When one difference per 100 bp is caused according to general genetics hypothesis, the amount of information to be recorded is equal to 1% of the amount of original information. Therefore, 1% of the whole human genome must be encoded. In conversion into a string of characters, eight characters (/100~1/1) per 100 bp must be further recorded, thereby causing a 8% increase in the amount of information to be recorded. Consequently, the amount of information to be recorded is equal to 9% of the amount of the original information. However, when the string of characters is expressed by 4 bit codes, the amount of information to be recorded is reduced in half. Finally, when the encoded information is compressed by a compression algorithm with a compression ratio of 70%, the amount of information to be recorded is equal to 1.35% of the amount of the original information. Therefore, when the whole human genome is compressed, a minimum compression ratio of 98.65% is theoretically ensured.

The present invention can be embodied as a computer readable code on a computer readable medium. The computer readable medium includes all types of recording medium storing data readable by computer system. For example, the computer readable medium includes ROMs, RAMs, CD-ROMs, magnetic tapes, floppy disks, optical data storage media, and carrier waves (e.g., transmissions over the Internet). Also, the computer readable medium may store computer readable codes distributed in computer systems connected by a network so that a computer can read and execute the codes in a distributed manner.

As is apparent from the above descriptions, according to an apparatus and a method for encoding a DNA sequence of the present invention, the DNA sequence can be compressed at a compression ratio of 90% or more without loss of genetic information and stored. Therefore, a genome sequence or multiple DNA sequences for a specific region of the genome can be stored. By way of an example, when individual specific disease genes derived from ten thousand patients who carry the genes are sequenced and stored, compression storage can decrease a storage space. Furthermore, the transfer speed and search efficiency of sequence data can be increased. Still furthermore, since only information of the difference between the DNA sequences is recorded, different DNA sequences can be efficiency compared and searched. For example, when there exist DNA sequences of ten thousand patients who carry a specific disease gene and normal persons, the sequence difference between the patients and normal persons or between the normal persons can be efficiently searched. Meanwhile, since a DNA sequence is encoded after modification of a reference sequence, security can be increased during storage and transfer of information on the DNA sequence. Also, since one or more of a plurality of reference sequences diversely modified are used as a secret key, higher security effect can be ensured.

While the present invention has been particularly shown and described with reference to exemplary embodiments thereof, it will be understood by those of ordinary skill in the art that various changes in form and details may be made therein without departing from the spirit and scope of the present invention as defined by the following claims.

**Claims**

1.  An apparatus for encoding a DNA sequence, which comprises:

    a comparative unit aligning a reference sequence having known DNA information with a subject sequence to be encoded and extracting a difference between the reference sequence and the subject sequence;
    a conversion unit converting information of the extracted difference between the reference sequence and the subject sequence into a string of predetermined characters;

a code storage unit storing predetermined conversion codes that correspond to the individual characters; and an encoding unit encoding the individual characters that make the string of the characters using the conversion codes.

2. The apparatus of claim 1, wherein the characters comprises a first character representing DNA base symbols, a second character representing the number of the difference, a third character representing the starting and ending of the difference, and a fourth character representing continuation of the difference.

3. The apparatus of claim 2, wherein the conversion unit converts respective information of starting, start position, continuation, the number of continued bases, bases, ending, and distance between the start position and the end position of the difference into the third character, the second character, the fourth character, the second character, the first character, the third character, and the second character, and outputs the string of the characters.

4. The apparatus of claim 1, wherein the difference comprises start region mismatch between the reference sequence and the subject sequence, blank by base deletion of the subject sequence corresponding to the reference sequence, single base pair mismatch between the reference sequence and the subject sequence, base insertion into the subject sequence, multiple base pair mismatch between the reference sequence and the subject sequence, and end region mismatch between the reference sequence and the subject reference.

5. The apparatus of claim 1, wherein the conversion codes are 4 bit codes, each of which corresponds to each of the characters.

6. The apparatus of claim 1, which further comprises a division unit dividing the extracted difference into segments of predetermined sizes, and
    wherein the conversion unit converts information of the extracted difference into the string of the characters based on the segments.

7. The apparatus of claim 1, which further comprises:

    a compression unit compressing the encoded subject sequence; and
    a sequence storage unit storing the compressed subject sequence.

8. The apparatus of claim 1, which further comprises a pre-processing unit creating a variation sequence generation factor from a variation sequence generation function that uses random variables as inputs and modifying the reference sequence using the created variation sequence generation factor.

9. The apparatus of claim 8, wherein the variation sequence induction factor comprises the total number of variations, distance between the variations, length of the variations, type of the variations, and a variation sequence.

10. A method for encoding a DNA sequence, which comprises:

    aligning a reference sequence having known DNA information with a subject sequence to be encoded;
    extracting a difference between the reference sequence and the subject sequence;
    converting information of the extracted difference between the reference sequence and the subject sequence into a string of predetermined characters; and
    encoding the individual characters that make the string of the predetermined characters using predetermined conversion codes that correspond to the individual characters.

11. The method of claim 10, wherein the characters comprises a first character representing DNA base symbols, a second character representing the number of the difference, a third character representing the starting and ending of the difference, and a fourth character representing continuation of the difference.

12. The method of claim 11, wherein converting comprises:

    allotting the third character for the starting of the difference;
    allotting the second character for the starting position of the difference;
    allotting the fourth character for the continuation of the difference;
    allotting the second character for the number of the continued bases of the difference;

allotting the first character for the bases of the difference;
allotting the third character for the ending of the difference;
allotting the second character for the distance between the start position and the end position of the difference; and
outputting the string of the allotted characters.

13. The method of claim 10, wherein the difference comprises start region mismatch between the reference sequence and the subject sequence, blank by base deletion of the subject sequence corresponding to the reference sequence, single base pair mismatch between the reference sequence and the subject sequence, base insertion into the subject sequence, multiple base pair mismatch between the reference sequence and the subject sequence, and end region mismatch between the reference sequence and the subject reference.

14. The method of claim 10, wherein the conversion codes are 4 bit codes, each of which corresponds to each of the characters.

15. The method of claim 10, which further comprises dividing the extracted difference into segments of predetermined sizes, and wherein in converting, information of the extracted difference is converted into the string of the characters based on the segments.

16. The method of claim 10, which further comprises:

compressing the encoded subject sequence; and
storing the compressed subject sequence.

17. The method of claim 10, which further comprises, before aligning, creating a variation sequence induction factor from a variation sequence induction function that uses random variables as inputs and modifying the reference sequence using the created variation sequence induction factor.

18. The method of claim 17, wherein the variation sequence induction factor comprises the total number of variations, distance between the variations, length of the variations, type of the variations, and a variation sequence.

19. A computer readable medium having embodied thereon a computer program for a method for encoding a DNA sequence, the method comprising:

aligning a reference sequence having known DNA information with a subject sequence to be encoded;
extracting a difference between the reference sequence and the subject sequence;
converting information of the extracted difference between the reference sequence and the subject sequence into a string of predetermined characters; and
encoding the individual characters that make the string of the characters using predetermined conversion codes that correspond to the individual characters.

# FIG. 1

REFERENCE
SEQUENCE

SUBJECT
SEQUENCE

COMPARATIVE
UNIT
110

DIVISION
UNIT
120

CONVERSION
UNIT
130

ENCODING
UNIT
140

CODE
STORAGE UNIT
160

SEQUENCE
STORAGE UNIT
170

COMPRESSION
UNIT
150

# FIG. 2

**REFERENCE SEQUENCE**

**SUBJECT SEQUENCE**

Color Key for Alignment Scores

<40    40-50

1_29318

0    500    1000    1500    2000    2500    3000

ref|NT_028327.5|Hs12_28406   Homo sapiens chromosome 12 refer...   2757   0.0

Alignments

>ref|NT_028327.5|Hs12_28406 Homo sapiens chromosome 12 reference genomic contig
Length = 906100

Score = 2757 bits (1434), Expect = 0.0
Identities = 1445/1450 (99%), Gaps = 2/1450 (0%)
Strand = Plus / Plus

Query: 1789   cagtgtcctccagcagcctggtgctgtaccagagctcagactccagcaatggccagagcc   1848
Sbjct: 367079  cagtgtcctccagcagcctggtgctgtaccagagctcagactccagcaatggccagagcc   367138

Query: 1849   acctgctgccatccaaccacagcgtcatcgagacgttcatctccacccagatggcctctt   1908
Sbjct: 367139  acctgctgccatccaaccacagcgtcatcgagacgttcatctccacccagatggcctctt   367198

Query: 1909   cctcccagtaaccacggcacctgggcacctggggacctgtactgcctgcttgggggtgatg   1968
Sbjct: 367199  cctcccagtaaccacggcacctgggcccctggggcctgtactgcctgcttgggggtgatg   367258

Query: 1969   aggggcagcagccagccctgcctggaggacctgagcctgccgagcaaccgtggccttcct   2028
Sbjct: 367259  aggggcagcagccagccctgcctggaggacctgagcctgccgagcaaccgtggccttcct   367318

Query: 2029   ggacagctgtgcctcgctccccactctgctcctgatgcatcagaaagggagggctctgagg   2088
Sbjct: 367319  ggacagctgtgcctcgctcccccactctgctcctgatgcatcagaaagggagggctctgagg   367378

Query: 2089   cgcccccaaccggtggaggctgctcggggtgcacaggagggggtcgtggagagctaggagc   2148
Sbjct: 367379  cgcccccaaccggtggaggctgctcggggtgcacaggagggggtcgtggagagctaggagc   367438

Query: 2149   aaagcctgttcatggcagatgtaggagggactgtcgctgcttcgtgggatacagtcttct   2208
Sbjct: 367439  aaagcctgttcatggcagatgtaggagggactgtcgctgcttcgtgggatacagtcttct   367498

http://www.ncbi.nlm.nih.gov/BLAST/

EP 1 443 449 A2

# FIG. 3

① Start region mismatch    ④ Multiple base pair mismatch
② Blank region             ⑤ Inserted region
③ Single base pair mismatch ⑥ End region mismatch

$X_0$       $X_6$      $X_{11}$   $X_{13}X_{14}$   $X_{16}$

| a | c | g | t | a | t | t | a | g | g | g | a | t | g | g | a | c | g | t | a | t | t |

$X_{-3}X_{-2}X_{-1}$ $X_0$     $X_6$     $X_{11}$   $X_{13}X_{14}$   $X_{16}$

| g | a | c | a | c | g | t | a | t |   | g | g | g | c | t | g | g | a | t | c | c | a | t | t | a | g |

| a | t | g | c | a | t |

/-3~3gac/3/6/2/3~1c/1/1~6atgcat/1/2~3tcc/3/4~2ag/2

# FIG. 4

4BITS CODES FOR ENCODING INFORMATIN ABOUT
THE DIFFERENCE BETWEEN DNA SEQUENCES

| | |
|---|---|
| 0 : 0000 | 8 : 1000 |
| 1 : 0001 | 9 : 1001 |
| 2 : 0010 | A : 1010 |
| 3 : 0011 | T : 1011 |
| 4 : 0100 | G : 1100 |
| 5 : 0101 | C : 1101 |
| 6 : 0110 | / : 1110 |
| 7 : 0111 | ~ : 1111 |

# FIG. 5

MODY3 exons (5552 characters = 5552 byte)
ref|NT_028327.5|Hs12_28486 Homo sapiens chromosome 12 reference genomic contig
Length = 906100

| | | |
|---|---|---|
| Exon2 /354759-1a/1 | Exon789 /363265-1t/1 | Exon789 /363384-1t/1 |
| Exon3 /359197-1t/1 | Exon789 /363261-1t/1 | Exon789 /363391-1t/1 |
| Exon456 /362119-1a/1 | Exon789 /363267-1t/1 | Exon789 /363394-1t/1 |
| Exon456 /362178-1a/1 | Exon789 /363273-1t/1 | Exon789 /363641-1a/1 |
| Exon789 /363208-1t/1 | Exon789 /363278-1t/1 | Exon789 /363689-1a/1 |

/354759-1a/1/4438-1a/1/2922-1a/1/59-1a/1/30-1a/53-1t/1/4-1t/1/2-1/t/1/6-1t/1/5-1t/1/6

1t/1/7-1t/1/3-1t/1/247-1a/1/248-1a/1

122byte

```
11100011010101000111010110011110000110101110000111100100010000111000111000011010111000011111000101001001000010
11100001101011100001111001011001111000011010111000011110001100001110000110101110010100111111000011011111100001
11100100111000011011111100001111000101110000111101011111100001111001101110000110111111000011111001011110000110 11
11100001111001101110000110111111000011110011111100001101111100001111000111110000110111110000111100010010001110
1110000110101110000111100010010010001110000110101110 0001
```

61byte

# FIG. 6

START

↓

EXTRACT DIFFERENCE BETWEEN
REFERENCE AND SUBJECT SEQUENCES — S600

↓

DIVIDE REFERENCE
INTO SIZES APPROPRIATE
TO BE PROCESSED IN MEMORY — S610

↓

CONVERT INFORMATION OF THE DIFFERENCE
BETWEEN REFERENCE AND SUBJECT
SEQUENCES INTO STRING OF CHARACTERS — S620

↓

ENCODE STRING OF CHARACTERS
USING 4 BIT CODES — S630

↓

COMPRESS ENCODED SUBJECT SEQUENCE — S640

↓

STORE COMPRESSED SUBJECT SEQUENCE — S650

↓

END

# FIG. 7

# FIG. 8

TOTAL NUMBER OF VARIATION:4

10000bp

Nd:1035    Nd:2200    Nd:3215    Nd:3200

10000bp

① 

DISTANE BETWEEN VARIATION
Nd : 1035

LENGTH OF VARIATION Lv : 1

TYPE OF VARIATION :
Substitution

VARIATION SEQUENCE : T

② 

DISTANE BETWEEN VARIATION
Nd : 2200

LENGTH OF VARIATION : 4

TYPE OF VARIATION :
Substitution

VARIATION SEQUENCE : ATGG

③ 

DISTANE BETWEEN VARIATION
Nd : 3215

LENGTH OF VARIATION : 7

TYPE OF VARIATION :
Insertion

VARIATION SEQUENCE :
ATGCGGG

④ 

DISTANE BETWEEN VARIATION
Nd : 3200

LENGTH OF VARIATION : 5

TYPE OF VARIATION :
Substitution

VARIATION SEQUENCE :
NULL NULL NULL NULL NULL

EP 1 443 449 A2

# FIG. 9

```
                    ( START )
                        │
                        ▼
┌─────────────────────────────────────────────┐
│    GENERATE VARIATION SEQUENCE GENERATION     │
│  FACTOR FROM VARIATION SEQUENCE GENERATION    │── S900
│          FUNCTION THAT USES AS INPUTS         │
│             RANDOM VARIABLES                  │
└─────────────────────────────────────────────┘
                        │
                        ▼
┌─────────────────────────────────────────────┐
│         MODIFY REFERENCE SEQUENCE USING       │
│  VARIATION SEQUENCE GENERATION FACTOR AND     │── S910
│     STORE MODIFIED REFERENCE SEQUENCE         │
└─────────────────────────────────────────────┘
                        │
                        ▼
┌─────────────────────────────────────────────┐
│       EXTRACT DIFFERENCE BETWEEN MODIFIED     │── S920
│       REFERENCE  AND SUBJECT SEQUENCES        │
└─────────────────────────────────────────────┘
                        │
                        ▼
┌─────────────────────────────────────────────┐
│           DIVIDE  DIFFERENCE  INTO SIZES      │── S930
│     APPROPRIATE TO BE PROCESSED IN MEMORY     │
└─────────────────────────────────────────────┘
                        │
                        ▼
┌─────────────────────────────────────────────┐
│       CONVERT INFORMATION OF THE DIFFERENCE   │
│   BETWEEN MODIFIED REFERENCE AND SUBJECT      │── S940
│     SEQUENCES INTO STRING OF CHARACTERS       │
└─────────────────────────────────────────────┘
                        │
                        ▼
┌─────────────────────────────────────────────┐
│          ENCODE STRING OF CHARACTERS          │── S950
│               USING 4 BIT CODES               │
└─────────────────────────────────────────────┘
                        │
                        ▼
┌─────────────────────────────────────────────┐
│       ENCRYPT ENCODED SUBJECT SEQUENCE        │── S960
└─────────────────────────────────────────────┘
                        │
                        ▼
┌─────────────────────────────────────────────┐
│      COMPRESS ENCRYPTED SUBJECT SEQUENCE      │── S970
└─────────────────────────────────────────────┘
                        │
                        ▼
┌─────────────────────────────────────────────┐
│       STORE COMPRESSED SUBJECT SEQUENCE       │── S980
└─────────────────────────────────────────────┘
                        │
                        ▼
                    (  END  )
```